# EUROPÄISCHE PATENTSCHRIFT

(11) **EP 1 808 156 B1**
(45) Veröffentlichungstag und Bekanntmachung des Hinweises auf die Patenterteilung: **08.06.2011**
(21) Anmeldenummer: 06026516.2
(22) Anmeldetag: 21.12.2006
(51) Int. Cl.: A61K 8/02, A61K 8/27, A61K 8/29, A61Q 17/04

(54) **Lichtschutzzubereitung mit Mikropigmenten**
Sun protection preparation with micro pigments
Préparation de protection contre la lumière avec micropigments

(30) Priorität: 22.12.2005 DE 102005062097
(43) Veröffentlichungstag der Anmeldung: 18.07.2007
(73) Patentinhaber: STADA ARZNEIMITTEL AG, 61118 Bad Vilbel (DE)
(72) Erfinder: Rillmann, Thomas, Dr., 76756 Bellheim (DE); Hansen, Peter, Dr., 63303 Dreiech (DE); Heppner, Andrea, 61197 Florstadt (DE)
(74) Vertreter: Hamm, Volker

(56) Entgegenhaltungen:
- EP-A- 1 579 847
- EP-A- 1 586 306
- EP-A1- 0 535 971
- EP-A1- 0 992 455
- WO-A-02/00797
- WO-A-03/041677
- WO-A-2004/108599
- POPOV, ALEXEY P. ET AL: "Effect of size of TiO2 nanoparticles embedded into stratum corneum on ultraviolet-A and ultraviolet-B sun-blocking properties of the skin" JOURNAL OF BIOMEDICAL OPTICS , 10(6), CODEN: JBOPFO; ISSN: 1083-3668, 8. Dezember 2005 (2005-12-08), Seiten 064037/1-064037/9, XP002435985
- "DIN 67502:2005-02. CHARAKTERISIERUNG DER UVA-SCHUTZWIRKUNG VON DERMALEN SONNENSCHUTZMITTELN DURCH TRANSMISSIONSMESSUNGEN UNTER BER]CKSICHTIGUNG DES LICHTSCHUTZFAKTORS" DEUTSCHE NORMEN. DIN NORM, XX, XX, Februar 2005 (2005-02), Seiten 1-10, XP009084219

## Beschreibung

Die Erfindung betrifft Lichtschutzzubereitungen mit einem UV-A-Bilanzwert >15, die als Filtersubstanzen ausschließlich anorganische Mikropigmente enthalten.

Es ist seit langem bekannt, dass die ultravioletten Teile des Sonnenlichts Schädigungen an der Haut hervorrufen. Diese Schädigungen können eine einfache Hautreizung, z.B. ein leichter Sonnenbrand sein, können sich aber auch in Zellschädigungen manifestieren. Es ist bekannt, dass vor allem durch UV-B-Strahlung (das ist Strahlung mit einer Wellenlänge zwischen 290 nm und 320 nm) DNS-Schäden hervorgerufen werden können, die zu Zellmutationen und somit zu Hautkrebs führen.

Auch werden die vorzeitige Hautalterung, die Bildung von Falten und die Erschlaffung des Hautbindegewebes durch Licht beschleunigt; verantwortlich hierfür ist vor allem die langwellige UV-A-Strahlung (das ist Strahlung mit einer Wellenlänge zwischen 320 nm und 400 nm).

Zur Vorbeugung gegen derartige Schädigungen sind in den letzten Jahren immer effektivere Sonnenschutzmittel entwickelt worden, wobei das Augenmerk insbesondere auf der Verbesserung des Schutzes gegen UV-B-Strahlung lag. Der UV-B-Schutz wird weltweit einheitlich als "Lichtschutzfaktor", abgekürzt LF oder SPF, angegeben, dessen Bestimmung in vielen nationalen Normen festgelegt ist. Moderne Sonnenschutzmittel erreichen einen Lichtschutzfaktor von über 30, der im allgemeinen durch eine Kombination verschiedener organischer UV-Filter mit reflektierenden Mikropigmenten erreicht wird.

Die Schutzwirkung eines Sonnenschutzmittels gegen UV-A-Strahlung wurde lange Zeit gemäß den Anforderungen der australischen Norm angegeben, die dann erfüllt sind, wenn ein Sonnenschutzmittel die Transmission im Bereich von 320-360 nm um mindestens 90% reduziert. Die australische Norm sieht oberhalb von 90% Absorption keine weitere Differenzierung vor, so dass bei Einhaltung der australischen Norm bei Produkten mit niedrigem Lichtschutzfaktor noch ein angemessener UV-A-Schutz sichergestellt ist, mit steigendem Lichtschutzfaktor der UV-A-Schutz aber auf gleichem Niveau stagnieren kann: Der UV-A-Schutz steigt nicht zwangsläufig mit einem steigenden Lichtschutzfaktor, der letztlich nur die Schutzwirkung gegen UV-B-Strahlung definiert.

Zur besseren Charakterisierung eines Sonnenschutzmittels hinsichtlich seiner UV-B- und UV-A-Schutzwirkung wurde der Begriff der UV-A-Bilanz eingeführt, der das Verhältnis zwischen der UV-B- und der UV-A-Schutzleistung eines Sonnenschutzmittels angibt. Das Verfahren zur Bestimmung des UV-A-Bilanzwertes ist in der DIN 67502 festgelegt.

Sonnenschutzmittel mit Mikropigmenten als anorganische UV-Filter sind seit langem bekannt. Die Mikropigmente wirken auf der Haut verteilt wie kleine Spiegel, die die UV-Strahlung reflektieren und streuen. Üblicherweise in Sonnenschutzmitteln verwendete Mikropigmente basieren auf Titandioxid oder Zinkoxid. Aufgrund ihrer Fotoreaktivität (die zu einer bläulichen Verfärbung von diese Pigmente enthaltenden kosmetischen Formulierungen führen kann) sind Titandioxid-Pigmente zur Stabilisierung bevorzugt mit einer hydrophoben oder amphiphilen Oberflächenbeschichtung versehen. Um ein "weißeln" auf der Haut zu vermeiden, werden Mikropigmente mit möglichst geringer Teilchengröße verwendet. So liegt die Teilchengröße der für kosmetische Anwendungen kommerziell erhältlichen Titandioxid-Pigmente aus der MT-Reihe der Firma Tayco beispielsweise bei 15 nm. Einige Hersteller von Mikropigmenten bieten ihre Produkte in Form von fertigen Dispersionen an, um sicherzustellen, dass im Endprodukt eine gleichmäßige feine Verteilung des Mikropigmentes erreicht wird, die für die optimale Lichtschutzwirkung erforderlich ist. Eine z.B. durch Agglomeration zunehmende Größe der Partikel führt zu einer erheblichen Senkung der UV-absorbierenden Wirkung.

Mikropigmente werden in Sonnenschutzmitteln zumeist in Kombination mit organischen Lichtschutzfiltern eingesetzt. Organische Lichtschutzfilter haben den Nachteil, dass sie zu Hautunverträglichkeiten führen können, die insbesondere bei Kindern beobachtet werden. Vorbekannte Produkte, die nur Mikropigmente als Lichtschutzfilter enthalten, haben den Nachteil geringer UV-A-Bilanzwerte von unter 15, oftmals unter 10. Der Nachteil dieser Produkte liegt in einem inadäquaten Schutz im UV-A-Bereich, insbesondere bei Produkten mit hohem UV-B-Lichtschutzfaktor.

Vor diesem Hintergrund ist es Aufgabe der vorliegenden Erfindung, Lichtschutzzubereitungen mit einem hohen UV-A-Bilanzwert sowie einem hohen Lichtschutzfaktor anzugeben, die sich durch eine sehr gute Hautverträglichkeit auszeichnen.

Gelöst wird diese Aufgabe dadurch, dass als Filtersubstanzen Mikropigmente mit einer mittleren Primär-Teilchengröße von mindestens 20 nm verwendet werden.

Überraschend wurde gefunden, dass bei Verwendung von bestimmten Pigmenten als Filtersubstanzen auch ohne die Verwendung von organischen Filtersubstanzen Sonnenschutzmittel hergestellt werden können, die einen hohen UV-B-Faktor und gleichzeitig einen hohen UV-A-Bilanzwert von mindestens 15 bis über 40 aufweisen. Die erfindungsgemäß verwendeten Pigmente haben eine mittlere Primär-Teilchengröße von mindestens 20 nm, vorzugsweise von 20-200 nm, und unterliegen ansonsten keinerlei Beschränkungen. So können sie auf Titandioxid oder Zinkoxid basieren und übliche Oberflächenbeschichtungen aufweisen. Besonders bevorzugt sind Titandioxidpigmente mit einer Primär-Teilchengröße von 10 nm, 15 nm, 20 nm, 35 nm, 60 nm oder 150 nm sowie Zinkoxidpigmente mit einer Primär-Teilchengröße von 20 nm, 60-100 nm oder 120 nm.

Die erfindungsgemäßen Pigmente können in Mengen von 1-50 Gew.-%, bezogen auf das Gewicht der sie enthaltenden Formulierung, enthalten sein. Bevorzugt sind Gehalte zwischen 5 und 40 Gew.-%, besonders bevorzugt Gehalte zwischen 10 bis 35 Gew.-%.

Die erfindungsgemäßen Lichtschutzzubereitungen können in üblicher Weise formuliert werden, z.B. als O/W-Emulsion, W/O-Emulsion, Hydrogel, Hydrodispersion oder auch in Form von wasserfreien Produkten.

Die O/W- bzw. W/O-Emulsionen umfassen grundsätzlich eine Ölphase, Wasser, ggf. Alkohol sowie mindestens ein anorganisches Mikropigment mit einer Teilchengröße von mindestens 20 nm. Die Ölphase kann dabei aus Fetten, Ölen, gelösten Wachsen oder sonstigen lipophilen Bestandteilen gebildet werden. Als Öle können vorteilhaft Paraffinöl, mittelkettige Triglyceride, wie beispielsweise Myritol 318, Octyldodecanol, Isopropylmyristat, Jojobaöl, Kokosnussöl oder Rizinusöl enthalten sein. Die wässrige Phase der erfindungsgemäßen Emulsionen wird üblicherweise aus Wasser, ggf. im Gemisch mit Alkohol gebildet.

Die erfindungsgemäßen Hydrodispersionen umfassen grundsätzlich eine Ölphase, ein geeignetes Verdickungsmittel, Wasser sowie einen wirksamen Gehalt an mindestens einem Mikropigment. Die Ölphase kann wiederum aus Fetten, Ölen, gelösten Wachsen oder anderen lipophilen Bestandteilen gebildet werden, wobei als Öle vorteilhaft die oben genannten Öle enthalten sein können.

Die Hydrogele gemäß der Erfindung umfassen neben dem Mikropigment grundsätzlich Wasser, ein geeignetes Verdickungsmittel sowie ggf. Alkohol.

Als Verdickungsmittel kommen für die Hydrodispersionsgele und Hydrogele alle üblicherweise verwendeten Verdickungsmittel in Betracht, bevorzugt werden jedoch Polyacrylsäure-Derivate, wie beispielsweise Polyacrylate aus der Gruppe der sog. Carbopole, Acrylsäurecopolymere, wie beispielsweise Pemulen TR1 oder Xanthan. Der Gehalt an Verdickungsmitteln in den erfindungsgemäßen Gelen beträgt 0,05-20 Gew.-%, bevorzugt 0,1-3 Gew.-% und besonders bevorzugt 0,5-1 Gew.-%.

Besonders bevorzugt sind die erfindungsgemäßen Zusammensetzungen frei von die Hautverträglichkeit potentiell negativ beeinflussenden Zusatzstoffen, insbesondere enthalten sie keine PEG-Emulgatoren, Konservierungsstoffe und Duftstoffe. Vorzugsweise sind die Zubereitungen wasserfest bzw. seewasserfest.

Neben Pigmenten mit einer Primär-Teilchengröße von 20-200 nm können die erfindungsgemäßen Lichtschutzformulierungen zusätzlich Pigmente mit kleineren Teilchengrößen enthalten.

Die folgenden Ausführungsbeispiele veranschaulichen erfindungsgemäße Zusammensetzungen, wobei die Inhaltsstoffe gemäß der INCI-Nomenklatur bezeichnet werden.

### Beispiel 1: W/O-Creme enthaltend Mikropigmente

| **Inhaltsstoff** | **Gehalt (Gew.-%)** |
|---|---|
| Polyglyceryl-2-Dipolyhydroxystearate | 3,0 |
| Glyceryl Oleate | 1,0 |
| Isopropylpalmitate | 5,0 |
| Magnesiumstearate | 1,0 |
| Titanium Dioxide 60 nm | 20,0 |
| Zinc Oxide 20 nm | 5,0 |
| Butylen Glycol | 5,0 |
| Disodium EDTA | 0,05 |
| Magnesium Sulfate | 0,7 |
| Sodium Lactate | 0,25 |
| Lactic Acid | 0,25 |
| Glycin | 1,2 |
| Alkohol Denat. | 4,0 |
| Tocopheryl Acetate | 0,5 |
| Wasser | ad 100,0 |

Isopropylpalmitate und Magnesiumstearate werden bei 120°C geschmolzen. Dann werden Polyglyceryl-2-Dipolyhydroxystearate, Glyceryl Oleate, Titanium Dioxide mit einer Primär-Teilchengröße von 60 nm, Zinc Oxide mit einer Primär-Teilchengröße von 20 nm sowie Tocopheryl Acetate zugegeben und bei 85°C geschmolzen. Parallel werden Butylen Glycol, Disodium EDTA, Magnesium Sulfate, Sodium Lactate, Lactic Acid und Glycin in Wasser gelöst und die beiden Ansätze vereinigt. Dann wird homogenisiert und unter Rühren auf 45°C abgekühlt. Bei 45°C wird unter Rühren Alkohol Denat. zugegeben, homogenisiert und unter Rühren auf 28°C abgekühlt.

Für die erhaltene Creme wurde nach der dem Fachmann bekannten COLIPA-Methode ein SPF von ca. 35 und gemäß der Methode nach DIN 67502 ein UV-A-Bilanzwert von ca. 27 bestimmt.

### Beispiele 2 und 3

Es wurden W/O-Cremes nach Beispiel 1 hergestellt, wobei jedoch zum einen 20 Gew.-% Titandioxid mit einer Primär-Teilchengröße von 20 nm und 5 Gew.-% Zinkoxid mit einer Primär-Teilchengröße von 60-100 nm (Beispiel 2) und zum anderen 20 Gew.-% Titandioxid mit einer Primär-Teilchengröße von 150 nm und 5 Gew.-% Zinkoxid mit einer Primär-Teilchengröße von 20 nm (Beispiel 3) verwendet werden.

Die für diese Formulierungen bestimmten SPF- und UV-A-Bilanzwerte sind in der nachfolgenden Tabelle zusammengefasst

**Tabelle 1**

| | **Beispiel 2** | **Beispiel 3** |
|---|---|---|
| SPF | ca. 40 | ca. 25 |
| UV-A-Bilanz | ca. 18 | ca. 40 |

## Patentansprüche

1. Verwendung von Mikropigmenten einer Primär-Teilchengröße von 20 nm bis 200 nm zur Erzielung eines UV-A-Bilanzwertes von mindestens 15 in Lichtschutzformulierungen, die frei von organischen Filtersubstanzen sind.

2. Verwendung nach Anspruch 1, **dadurch gekennzeichnet,**
**dass** die Mikropigmente anorganische Mikropigmente sind.

3. Verwendung nach den Ansprüchen 1 oder 2, **dadurch gekennzeichnet,**
**dass** die Mikropigmente Titandioxid und/oder Zinkoxid sind.

4. Verwendung nach einem der Ansprüche 1, 2 oder 3, **dadurch gekennzeichnet,**
**dass** die Mikropigmente mit einer Primär-Teilchengröße von 20 bis 200 nm in Mengen von 1-50 Gew.-%, bevorzugt 5-40 Gew.-% und besonders bevorzugt 10-35 Gew.-% in der Lichtschutzformulierung enthalten sind.

5. Verwendung nach einem der Ansprüche 1, 2, 3 oder 4, **dadurch gekennzeichnet,**
**dass** der UV-A-Bilanzwert zwischen 15 und 40 liegt.

## Claims

1. Use of micropigments having a primary particle size of from 20 nm to 200 nm in order to achieve a UV-A balance value of at least 15 in sun protection formulations that are free of organic filter substances.

2. Use according to claim 1, **characterised in that** the micropigments are inorganic micropigments.

3. Use according to claim 1 or 2, **characterised in that** the micropigments are titanium dioxide and/or zinc oxide.

4. Use according to any one of claims 1, 2 or 3,
**characterised in that**
the micropigments having a primary particle size of from 20 to 200 nm are present in the sun protection formulation in amounts of from 1 to 50 wt.%, preferably from 5 to 40 wt.% and particularly preferably from 10 to 35 wt.%.

5. Use according to any one of claims 1, 2, 3 or 4,
**characterised in that**
the UV-A balance value is from 15 to 40.

## Revendications

1. Utilisation de micropigments dont la taille des particules primaires est comprise entre 20 nm et 200 nm pour obtenir un bilan UVA d'une valeur minimum de 15 dans des préparations de protection solaire exemptes de substances filtrantes organiques.

2. Utilisation selon la revendication 1, **caractérisée en ce que** les micropigments sont des micropigments minéraux.

3. Utilisation selon la revendication 1 ou la revendication 2, **caractérisée en ce que** les micropigments sont du dioxyde de titane et/ou de l'oxyde de zinc.

4. Utilisation selon l'une des revendications 1, 2 ou 3, **caractérisée en ce que** les micropigments dont la taille des particules primaires est comprise entre 20 et 200 nm sont présents à hauteur de 1 à 50 % en poids, de préférence 5 à 40 % en poids, et de manière particulièrement préférée 10 à 35 % en poids de la préparation de protection solaire.

5. Utilisation selon l'une des revendications 1, 2, 3 ou 4, **caractérisée en ce que** le bilan UVA a une valeur comprise entre 15 et 40.
